Europäisches Patentamt

European Patent Office

Office européen des brevets

(11) Publication number: **0 261 738**
**A1**

## (12) EUROPEAN PATENT APPLICATION

(21) Application number: **87201812.2**

(22) Date of filing: **22.09.87**

(51) Int. Cl.⁴: **A23L 3/22** , A23C 3/033

(30) Priority: **23.09.86 NL 8602400**

(43) Date of publication of application:
**30.03.88 Bulletin 88/13**

(84) Designated Contracting States:
**AT BE CH DE ES FR GB GR IT LI LU NL SE**

(71) Applicant: STORK AMSTERDAM B.V.
2 Ketelstraat
NL-1021 JZ Amsterdam(NL)

(72) Inventor: Meijer, Adam
Prof. Pieter Brandsmalaan 19
NL-3351 XZ Vleuten(NL)

(74) Representative: van der Veken, Johannes
Adriaan et al
EXTERPATENT B.V. Willem Witsenplein 4
NL-2596 BK 's-Gravenhage(NL)

(54) Method for performing a heat treatment of a product flowing through a heat-treatment installation of the through-flow type.

(57) In a method for performing a heat treatment of a product flowing through a heat-treatment installation of the through-flow type, the product to be treated is heated indirectly by a heating medium. During the heat treatment, in which contamination of the installation occurs, the value of a theoretical parameter which is a measure for the heat-treatment effect of the product heated, is continuously determined and regulated directly by means of adjustment of the temperature of the heating medium. If the theoretical parameter which is a measure for the heat-treatment effect reaches a certain minimum value as a consequence of increased contamination of the installation, the installation is temporarily shut down and is cleaned.

FIG. 3.

operating time

switch off installation on reaching F min.

EP 0 261 738 A1

## Method for performing a heat treatment of a product flowing through a heat-treatment installation of the through-flow type.

The invention relates to a method for performing a heat treatment of a product flowing through a heat-treatment installation of the through-flow type, the product to be treated being heated indirectly by a heating medium and, during the heat treatment, in which contamination of the installation occurs, the value of a certain parameter related to the product treated being regulated by means of adjustment of the temperature of the heating medium, and, of said parameter reaches a certain minimum value as a consequence of increased contamination of the installation, the installation being temporarily shut down and being cleaned.

In general, a first main effect is aimed at in a heat treatment of a product, but, as a result of the heat treatment, a number of less desirable side effects may occur. In order to achieve the desirable main effect and, at the same time, to avoid the undesirable side effects as well as possible, and in order, furthermore, to produce as uniform a treatment product as possible during the operation of the installation, it is, in general, of importance to maintain a closely toleranced combination of the time in which the product is at a certain temperature and the level of said temperature. Thus, for example, in the sterilization of milk, the degree of sterilization should not fall below a certain value, whereas, at the same time, the colour, taste and other quality changes occurring during the sterilization as a result of the milk being held for a certain time at elevated temperature have to be limited.

The effect of the heat treatment on the product can be expressed in a certain theoretical parameter which can be calculated from the temperature curve and the treatment time of the product, and the value of which increases as the treatment temperature and/or the treatment time increases.

In the foodstuffs industry, strict requirements are imposed by the authorities on the reproducibility and the reliability of the processing and on the good quality of the installations used, in order to be able to guarantee that the treated products are not harmful to public health. For heat-treatment installations of the abovementioned type with indirect heating it has therefore to be certain that the heat-treatment effect on the product treated therein does not drop below a minimum value, which implies that the theoretical parameter corresponding thereto should always be higher than a fixed minimum value. The installations consist in general of sections in which the product is consecutively preheated, heated to a maximum temperature, held for some time at said temperature and then cooled again. During operation, however, deposits from the product occur in the installation which increase with time, as a result of which, on the one hand, the heat transfer through the heat-exchange surfaces reduces, while, on the other hand, the passages for the product become smaller and, as a result thereof, the flow rates become larger so that the residence times decrease. As a result of this the heat-treatment effect decreases during operation under otherwise constant operating conditions. In order to be assured of the fact that the minimum required heat-treatment effect is still reached at the end of the operating time, the dimensioning of modern installations is such that under normal operating conditions a higher heat-treatment effect can be reached than is required. It is usual to operate such installations as follows.

At normal production flow rate, the parameters of the heating medium, usually stem, are regulated in a manner such that the product is heated in the heating section to a temperature such that, with the resulting residence time of the product, a higher heat-treatment effect than the required value is achieved. With contamination of the installation increasing with the course of time, increasing resistances to the heat transfer through the heat exchanging surfaces are produced, which, if no measures are taken, will reveal itself in a drop in the heating temperature of the product. Said temperature is measured and serves as control signal for raising the pressure of the heating medium steam and consequently of the heating temperature, as a result of which the temperature difference between the heating medium and the product becomes larger and the heating temperature of the product can be maintained. Since, if the installatioin is contaiminated, the residence time of the product will decrease, as already stated earlier if the same flow rate is maintained, the heat-treatment effect will decrease. From the instant the maximum available pressure and, consequently, temperature of the heating medium steam is reached, the temperature differenct between steam and product will not be able to rise further and, if processing is continued, the heating temperature of the product will start to fall and, consequently so will the heat-treatment effect to an accelerated extent because both the temperature and the residence time decreases. When the temperature drops below a certain set minimum temperature, the installation is switched off because otherwise the heat-treatment effect would be too small. The installation then has to be cleaned.

The known method for performing a heat treatment has, as a first disadvantage, that a product is delivered which has a variable heat-treatment effect and a variable quality. At the beginning, the heat-treatment effect is higher than necessary and the quality of the product is poorer as a result of an intensified occurrance of colour, taste and other quality changes as a result of temperatures which are in fact too high, with the occurring relatively long residence times. After the elapse of time, the heat-treatment effect approaches the required value and the quality of the product becomes better.

A second disadvantage is that, as a result of the heating temperature which is in fact unnecessarily high, an unnecessarily rapid contamination occurs at the start of the process and this unfavourably effects the operating time of the installation.

The object of the invention is therefore to provide a method as a result of which said disadvantages are eliminated and the heat treatment results in a product with a desired, in particular, constant heat-treatment effect and as high as quality as possible.

This object is achieved according to the invention by a method of the type described in the introduction, which is characterized in that, during the heat treatment, the value of a theoretical parameter which is a measure for the heat-treatment effect of the product treated is continuously determined and is regulated directly.

In this manner the result can be achieved that the heating temperature of the product at the beginning of the process can be kept lower than in the known method so that the quality of the product is better, in particular at the beginning of the process, than in the known method, while at the end of the operating time the heating temperature of the product and the product quality are virtually the same as in the known method. In addition, the operating time can be prolonged as a result of the fact that, at the beginning of the process, the heating temperature of the product of which the contamination is also less than in the known methods.

Expediently, the theoretical parameter which is a measure for the heat-treatment effect is regulated to a constant value.

The value of the theoretical parameter which is a measure for the heat-treatment effect is preferably calculated from the temperature and the residence time of the product treated in the segment of the installation where the heat treatment takes place.

If the installation comprises different sections which the product flows through and in which different phases of the heat treatment takes place, the value of the theoretical parameter which is a measure for the heat-treatment effect is obtained by summing the subvalues of the theoretical parameter calculated for the different sections.

The residence time of the product in a segment or a section of the installation can be determined on the basis of the measured pressure difference over the segment considered or the section considered and/or from the total heat transfer coefficient of the segment considered or the section considered calculated from the measured flow rate and the temperature.

In this manner, as a result of measuring the temperatures, pressures and the prevailing product flow rate and of performing various calculations, the theoretical parameter is determined which is a measure for the heat-treatment effect.

A pratical embodiment of the method according to the invention is characterized in that the heat treatment is started in an uncontaminated installation with a heating temperature of the treated product such that the desirable heat-treatment effect is achieved, during the heat treatment the value of the theoretical parameter which is a measure for the heat-treatment effect is continuously calculated and compared with the desired value, the set value of the heating temperature of the product in the installation being adjusted on the basis of the difference between the calculated value and the desired value in a manner such that the heat-treatment effect remains essentially constant and the installation is temporarily shut down and is cleaned when the maximum temperature of the heating medium is reached and as a consequence of increasing contamination of the installation, the heating temperature and the residence time of the product in the segment or the sections of the installation in which the heat treatment takes place has dropped to such an extent that the theoretical parameter which is a measure for the heat-treatment effect reaches the predetermined minimum value.

The invention will now be explained in more detail on the basis of an exemplary embodiment by means of the drawings, in which

Figure 1 is a diagram of a heat-treatment installation of the through-flow type,

Figure 2 is a graph which shows the variation of the various parameters in performing the known method, and

Figure 3 is a graph which shows the variation of the various parameters in performing the method according to the invention.

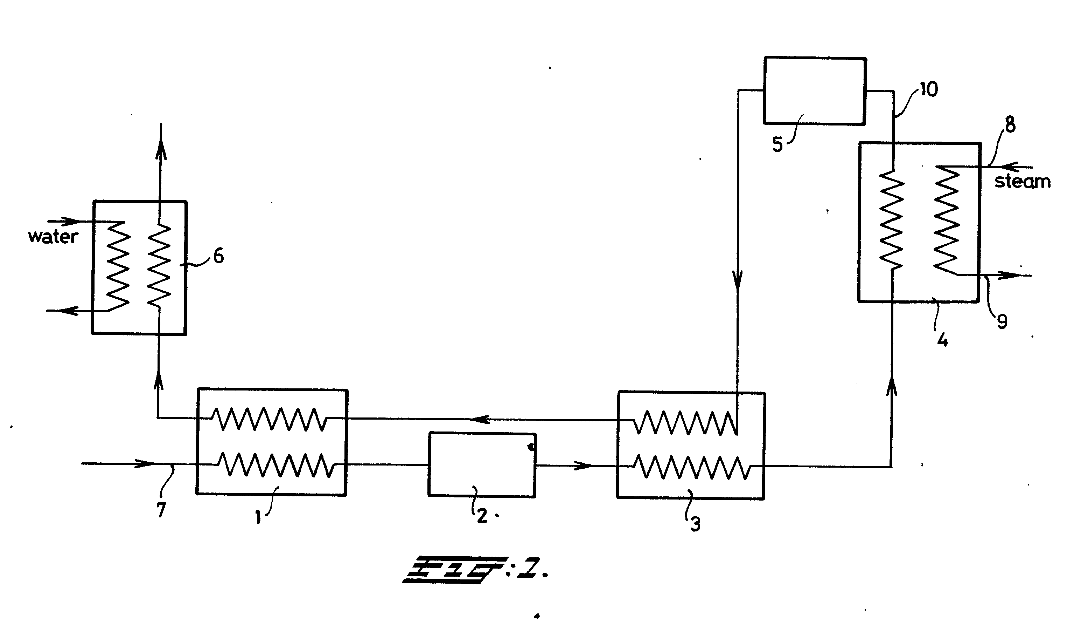

The heat-treatment installation shown in Figure 1 comprises a first regenerative unit 1, a homogenizer 2, a second regenerative unit 3, a heating unit 4, a heat-retaining unit 5 and a colling unit 6. The product to be treated is fed in at 7 to the first regenerative unit 1, where it is heated by heat exchange with the treated product. From the first regenerative heating unit 1, the product flows to the homogenizer 2, which comprises a pump with a positive displacement and a homogenizing element. Said pump pumps a predetermined quantity of product per unit of time through the installation.

From the homogenizer, the product is passed to the second regenerative unit 3 where it is further heated by heat exchange with product originated from the heat-retaining unit 4. From the second regenerative unit, the product is fed to the heating unit. In the heating unit 4, the product is heated with a heating medium, for example steam, which is fed into the unit at 8 and leaves the unit again as condensed water at 9. Here the product is heated to the heating temperature. Subsequently, the product is held at this temperature for some time in the heat-retaining unit 5.

Subsequently, the treated product is passed consecutively through the regenerative unit 3 and the regenerative unit 1, where the abovementioned heat exchange takes place with the product to be treated which is being fed in. Finally, the treated product is passed through a cooler 6, where it is cooled with water, and from there to a bottling unit or a storage tank.

In the operation of the installation according to the known method, the heating temperature $T_p$ of the treated product is measured at 10. Said temperature is regulated by regulating the steam pressure $P_s$ at the position of the point 8 and the steam temperature $T_s$ coupled therewith in the heating unit 4. During the heat treatment if the product deposits occurred in the installation as a result of which, on the one hand, the heat transfer in the various units, in particular in the second regenerative unit 3 and the heating unit 4, will fall, while, on the other hand, the passage available for the product will decrease. Since the flow rate of the product to be treated is kept constant, the flow rate through the installation will increase and as a result of this, the residence time of the product to be treated will drop in the various units. The heating temperature $T_p$ of the product at the position of the point 10 will exhibit the tendency to fall. The fall in the heating temperature $T_p$ is, however, compensated for by an increase in the steam temperature $T_s$ in a manner such that the heating temperature remains essentially constant. Afer a prolonged operating period, the steam pressure $P_s$, and consequently the steam temperature $T_s$, will, however, reach the maximum possible value, as a result of which, if the process is continued further, and consequently the contamination of the installation increases, the heating temperature $T_p$ will fall. At the instant when the heating temperature $T_p$ reaches the set minimum value, the treatment should be stopped and the installation should be cleaned.

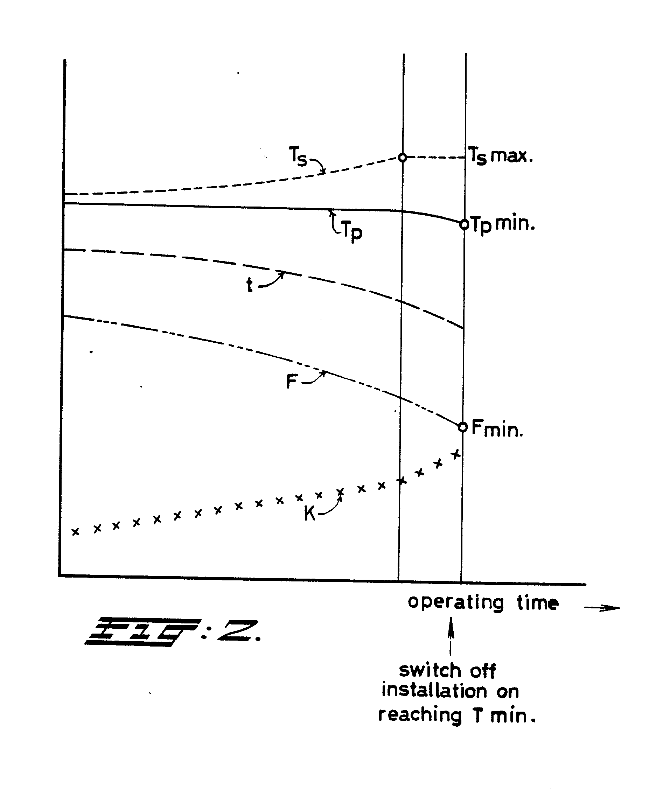

Figure 2 shows the variation of the various parameters during the process. In addition to the curve of the abovementioned parameters such as the residence time t, the heating temperature $T_p$ and the steam temperature $T_s$, the variation of the theoretical parameter F, which is a measure for the heat-treatment effect, and the product quality K are of importance. As a result of the fall in the residence time t for a constant heating temperature $T_p$, the value of the theoretical parameter which is a measure for the heat-treatment effect will fall from the start of the process. The product quality, on the other hand, will rise because the product is exposed for a shorter time to a certain temperature. The heating temperature $T_p$ is kept constant at a value such that, at the instant, when the temperature $T_s$ has reached its maximum value, the heat treatment effect for the residence time which is then applicable is still adequate to allow the treated product to meet the requirements with respect to, for example, sterility. This implies that the heating temperature is kept constant at a value which is relatively too high, which means that, in particular at the beginning of the process when the residence time t is still relatively large, the product quality will be relatively low as a result of the combination of high temperature and long residence time.

In performing the method according to the invention it is not the heating temperature $T_p$ of the product but the value of the theoretical parameter F which is a measure for the heat-treatment effect which is essentially kept constant. Said theoretical parameter F is a function of the temperature and the residence time of the product in the segment of the installation in which the heat treatment takes place. On increasing the temperature and prolonging the residence time, the value of the theoretical parameter F will increase.

If the installation comprises different sections which the product flows through and in different phases of the heat treatment take place, the theoretical paramter F is a function of the temperature and the residence time of the product in each of the different sections.

Theoretically, it can be deduced that, for a certain flow rate of the product stream through a pipe, for example a heat exchanged, there is a relationship between the residence time of the product in the pipe section and the pressure drop across the pipe section considered. This means that the value or subvalue of the theoretical parameter F which is a measure for the heat-treatment effect for the segment or the section of the installation in which the heat treatment or a phase thereof takes place can be calculated from the pressure drop across, and the temperature of the treated product in, the segment considered or the section considered of the installation.

In order, therefore, to achieve a certain value of the parameter F which is a measure for the heat-treatment effect at a certain flow rate of the product stream at any instant in time, the current value thereof should be determined. For this purpose, the pressure drop across the segment or the sections of the installation in which the heat treatment takes place is measured and the residence time or times are calculated therefrom. At the same time, the temperature in the segment considered or the sections considered is measured. From the measured value, the value of the thereoretical parameter F is calculated. In the event that the installation consists of different sections, the total value of the theoretical parameter F is determined by summing the calculated subvalues of the thereotical parameter for the various sections.

The calculated current value of the theoretical parameter F is compared with the desired value, and the said value of the heating temperature Tp of the product in the heating unit 4 is adjusted on the basis of the observed difference between the current value and the desired value. In this connection it should be pointed out that all the product temperatures in the installation are dependent on the heating temperature Tp of the product and rise or fall with the temperature Tp.

The required heating temperature Tp is then reached by automatically adjusting the steam pressure Ps and the steam temperature Ts, as in the known method.

It is, however, also possible to control the temperature of the heating medium (steam) directly on the basis of the observed difference between the current value and the desired value of the theoretical parameter F.

The value of the total heat-transfer coefficient of the segment considered or the section considered can be calculated from the values of the product flow rate and the measured temperatures in a segment or a section of the installation. From this it is then also possible to calculate the thickness of the deposition layer and, in addition, the residence time of the product. This provides a check on the thickness of the deposition layer and residence time calculated from the pressure difference measurement.

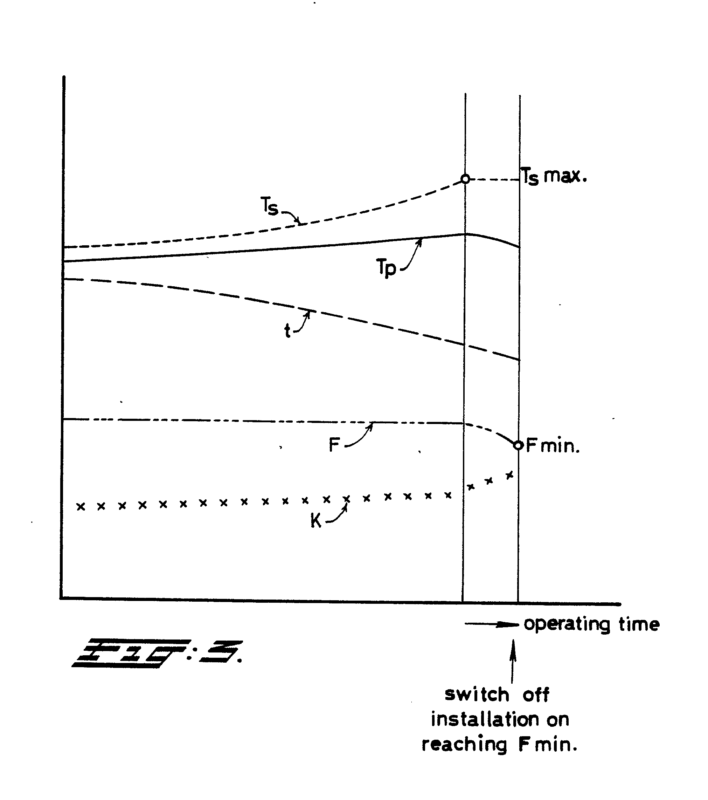

Figure 3 shows the variation of the different parameters in the method according to the invention. Here it can be seen that the heat-treatment effect F remains constant until the instant when the steam temperature Ts has reached its maximum value. As a result of contamination of the installation, the residence time t falls and the heating temperature Tp increases. When the steam temperature has reached its maximum possible value, the heat-treatment effect will fall as a result of the fall in the heating temperature Tp. When the heat-treatment effect has reached the limit blow which it must not fall, the installation is switched off in order to be cleaned.

From the beginning of the process, the product quality gradually becomes somewhat higher. The rise is, however, markedly less than in the known method. At the beginning of the process the product quality is much higher than in the known process.

The advantage of the method according to the invention over the known method lies in the fact that a product is produced having a constant heat-treatment effect and a high quality which is higher when viewed as a whole than in the known method as a result of the fact that the heating temperature of the product is not higher than necessary. In addition the contamination in this process will proceed more slowly as a result of the fact that the operating time between the beginning of the process and the instant at which the installation has to be cleaned is prolonged. In short, a product is produced which has a better quality with a more economical processing.

## Claims

1. Method for performing a heat treatment of a product flowing through a heat-treatment installation of the through-flow type, the product to be treated being heated indirectly by a heating medium and, during the heat treatment, in which contamination of the installation occurs, the value of a certain parameter related to the product treated being regulated by means of adjustment of the temperature of the heating medium, and, if said parameter reaches a certain minimum value as a consequence of increased contamination of the installation, the installation being temporarily shut down and being cleaned, **characterized in that**, during the heat treatment, the value of a theoretical paramter which is a measure for the heat-treatment effect of the product treated is continuously determined and is regulated directly.

2. Method according to claim 1, **characterized in that** the theoretical parameter which is a measure for the heat treatment effect is regulated to a constant value.

3. Method according to claim 1 or 2, **characterized in that** the value of the theoretical parameter which is a measure for the heat-treatment effect is calculated from the temperature and the residence time of the product treated in the segment of the installation where the heat treatment takes place.

4. Method according to one of the claims 1-3, **characterized in that**, if the installation comprises different sections which the product flows through and in which different phases of the heat treatment take place, the value of the theoretical parameter which is a measure for the heat treatment effect is obtained by summing the subvalues of the theoretical parameter calculated from the different sections.

5. Method according to claim 3 or 4, **charaterized in that** the residence time of the product in a segment or a section of the installation is determined on the basis of the measured pressure difference over the segment considered or the section considered.

6. Method according to claim 3 or 4, **characterized in that** the residence time of the product in a segment or section of the installation is determined from the total heat-transfer coefficient of the segment considered or the section considered calculated from the measured flow rate and the temperatures.

7. Method according to one or more of the claims 1-6, **characterized in that** the heat treatment is started in an uncontaminated installation with a heating temperature of the treated product such that the desirable heat-treatment effect is achieved, during the heat treatment the value of the theoretical paramter which is a measure for the heat treatment effect is continuously calculated and compared with the desired value, the set value of the heating temperature of the product in the installation being adjusted on the basis of the difference between the calculated value and the desired value in a manner such that the heat-treatment effect remains essentially constant and the installation is temporarily shut down and is cleaned when the maximum temperature of the heating medium is reached and as a consequence of increasing contamination of the installation, the heating temperature and the residence time of the product in the segment or the section of the installation in which the heat treatment takes place has dropped to such an extent that the theoretical parameter which is a measure for the heat-treatment effect reaches the predetermined minimum value.

Fig.2.

Fig: 2.

FIG. 3.

Ts max.

Ts

Tp

t

F min.

F

K

operating time

switch off
installation on
reaching F min.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | DE-A-2 929 823 (E.I. AFANASIEVITSCH et al.) <br> * Page 39, paragraph 2; claim 1; page 43, paragraphs 3,4 * | 1-3,7 | A 23 L 3/22 <br> A 23 C 3/033 |
| Y | FR-A-2 375 900 (STORK) <br> * Page 2, lines 20-33; page 3, lines 1-22; claims 1-3; figures 1,2 * & NL-A-76 14 605 | 1-3 | |
| Y | EP-A-0 118 134 (STORK) <br> * Claims 1,3,6; page 9, lines 1-11,20-31; page 10, lines 5-10 * | 1-3,7 | |
| Y | EP-A-0 081 256 (STORK) <br> * Page 3, lines 16-27; claims 1-5 * | 1,2 | |
| Y | GB-A-2 131 673 (A.P. MILBURN) <br> * Page 2, lines 30-54; page 2, lines 92-112; claims 1,10-12 * | 1-7 | |
| A | US-A-2 522 796 (R.E. OLSON et al.) <br> * Column 1, lines 42-54; column 3, lines 40-70; column 5, line 26 - column 6, line 73; claim 1 * | 1,5 | **TECHNICAL FIELDS SEARCHED (Int. Cl.4)** <br><br> A 23 L <br> A 23 C |
| Y | US-A-2 214 175 (R.E. OLSON) <br> * Claim 1; page 3, lines 25-37,56-75 * | 1,7 | |
| Y | FR-A-2 454 141 (M. LALANDE et al.) <br> * Page 3, line 1 - page 5, line 14; page 6, line 25 - page 7, line 28; figures 2-5 * | 1-7 | |

The present search report has been drawn up for all claims

| Place of search | Date of completion of the search | Examiner |
|---|---|---|
| THE HAGUE | 22-12-1987 | GUYON R.H. |

EPO FORM 1503 03.82 (P0401)